Europäisches Patentamt

European Patent Office

Office européen des brevets

(19)

(11) Veröffentlichungsnummer : **0 047 831**
**B1**

(12) **EUROPÄISCHE PATENTSCHRIFT**

(45) Veröffentlichungstag der Patentschrift :
15.05.85

(21) Anmeldenummer : 81105439.4

(22) Anmeldetag : 11.07.81

(51) Int. Cl.⁴ : **C 07 C 67/38**, B 01 J 31/28,
B 01 J 31/40

(54) Verfahren zur Herstellung von überwiegend linearen aliphatischen Carbonsäureestern.

(30) Priorität : 12.09.80 DE 3034422

(43) Veröffentlichungstag der Anmeldung :
24.03.82 Patentblatt 82/12

(45) Bekanntmachung des Hinweises auf die Patenterteilung : 15.05.85 Patentblatt 85/20

(84) Benannte Vertragsstaaten :
AT BE FR GB IT NL

(56) Entgegenhaltungen :
EP-A- 0 008 024
EP-A- 0 021 010
EP-A- 0 038 919
DE-A- 2 503 996
DE-A- 2 912 489
Patents Abstracts of Japan, Band 5, Nr. 12, 24.
Januar 1981

(73) Patentinhaber : **CHEMISCHE WERKE HÜLS AG**
**Postfach 1320**
**D-4370 Marl 1 (DE)**

(72) Erfinder : **Hofmann, Peter, Dr.**
**Lipper Weg 193**
**D-4370 Marl (DE)**
Erfinder : **Müller, Wolfgang Hans Eduard, Dr.**
**Bitterfelder Strasse 9a**
**D-4370 Marl (DE)**

EP 0 047 831 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Es ist bekannt, daß man durch Umsetzung von Olefinen mit Kohlenmonoxid und einer H-aciden Komponente, wie z. B. Alkanolen, in Gegenwart eines Katalysators, der ein Metall der 8. Nebengruppe des Periodischen Systems der Elemente und gegebenenfalls einen Promotor enthält, Fettsäureester herstellen kann (J. Falbe, Synthesen mit Kohlenmonoxid, Springer-Verlag, Berlin, Heidelberg, New York 1967).

Eine besonders bevorzugte Variante dieser als Hydrocarboxilierung bezeichneten Reaktion ist die Umsetzung in Gegenwart von kobalthaltigen Katalysatoren. Geschwindigkeit, Selektivität sowie die Ausbeute an linearen Fettsäureestern der kobaltkatalysierten Reaktion können durch die Zugabe von Promotoren, die zur Verbindungsklasse der Pyridine gehören, erhöht werden. Als besonders wirksam haben sich Pyridin selbst sowie nicht-ortho-substituierte Alkylpyridine erwiesen.

Ein wesentlicher Nachteil für die Benutzung solcher Promotoren ergibt sich aus dem relativ hohen Preis für Pyridin und nicht-ortho-substituierte Alkylpyridine sowie aus der nur in wenigen Fällen gegebenen Verfügbarkeit dieser Verbindungen in technischen Mengen.

Neben den Kosten für das als Promotor eingesetzte Pyridin bzw. nicht-ortho-substituierte Alkylpyridin wird die Wirtschaftlichkeit eines Hydrocarboxilierungsverfahrens u. a. auch durch die erzielbare Esterselektivität und durch die Verwertbarkeit der Nebenprodukte beeinflußt.

Während die Verwertung des als Nebenprodukt durch Olefinhydrierung gebildeten Paraffins bzw. des durch Hydroformylierung entstandenen Aldehyds im allgemeinen keine Schwierigkeiten bereitet — Paraffine können z. B. erneut zur Herstellung von Ausgangsolefin eingesetzt werden ; Aldehyde sind ebenso wie Ester für die Herstellung von Alkohol bzw. Carbonsäure verwendbar — hat man bis heute noch keine wirtschaftlich sinnvolle Verwertung für das als Nebenprodukt anfallende Stoffgemisch, das höher siedet als die als Reaktionsprodukt gebildeten Ester und das als Katalysatorbestandteil eingesetzte Pyridin und/oder nicht-ortho-substituierte Alkylpyridin (Hochsieder), gefunden.

Um eine Anreicherung dieser im allgemeinen — bei einer destillativen Aufarbeitung des Hydrocarboxilierungsgemisches — zusammen mit dem Katalysator als Destillationssumpf anfallenden Hochsieder bei wiederholter oder kontinuierlicher Durchführung der Reaktion zu vermeiden, werden sie gemäß den Verfahren des Standes der Technik entweder durch aufwendige Aufarbeitungsverfahren vom Katalysatormetall agbetrennt und verworfen (EP-OS 0 008 024 ; DE-PS 921 988) oder zusammen mit dem Katalysator verbrannt (DE-OS 27 45 377 ; US-PS 4 041 057).

Die DE-OS 25 03 996 und EP-A-38919 beschreiben zwar ebenfalls die Aufarbeitung von durch Hydrocarboxilierung erhaltenen Reaktionsgemischen. Angaben über den Verbleib und die Nutzung hierbei anfallender Hochsieder werden jedoch nicht gemacht. Zudem unterscheidet sich das Verfahren gemäß DE-OS 25 03 996 von dem erfindungsgemäßen Verfahren durch die zwingend vorgeschriebene Verwendung von Vinylpyridin als zusätzliche Promotorkomponente.

Aufgabe der vorliegenden Erfindung war es, ein Verfahren zur Herstellung von überwiegend linearen aliphatischen Carbonsäureestern zu entwickeln, das

1. unter Bewahrung der Vorteile der mit Kobalt und Pyridin und/oder Pyridinderivaten katalysierten Hydrocarboxilierung nicht mit den hohen Kosten für die Bereitstellung der Promotoren belastet ist und

2. eine wirtschaftlich sinnvolle Verwertung der bei der mit Kobalt und Pyridin und/oder Pyridinderivaten katalysierten Hydrocarboxilierung gebildeten hochsiedenden Nebenprodukte gestattet.

Diese Aufgabe wurde überraschend durch die in den Patentansprüchen beschriebenen Maßnahmen gelöst. Überraschend deshalb, weil nach dem in den obengenannten Patentschriften offenbarten Stand der Technik nicht zu erwarten war, daß die Hochsieder eine den üblicherweise verwendeten pyridinischen Promotoren vergleichbare cokatalytische Wirkung entfallen.

Das erfindungsgemäße Verfahren kann im Prinzip bei allen Hydrocarboxilierungsreaktionen, die in Gegenwart eines kobalthaltigen Katalysators durchgeführt werden, angewandt werden (z. B. Verfahren gemäß US-PS 3 507 891 und deutscher Patentanmeldung P 29 12 489.8). So ist vor allem die Wahl des eingesetzten Olefins unkritisch, d. h. es können sowohl geradkettige oder verzweigte α-Olefine als auch Olefine mit innenständiger Doppelbindung eingesetzt werden. Aber auch Olefine mit mehr als einer Doppelbindung und solche mit Substituenten, wie z. B. Aryl-, Cyano-, Carboximethyl- und Hydroxylgruppen, sind geeignet.

Im allgemeinen werden Olefine mit 2 bis 40, vorzugsweise 4 bis 20 Kohlenstoffatomen, eingesetzt, die nach bekannten Verfahren des Standes der Technik erhalten werden können. So können z. B. α-Olefine durch Oligomerisierung von Ethylen nach Ziegler (DE-PSS 878 560 und 11 90 930) oder durch Wachscracken, Olefine mit innenständiger Doppelbindung durch katalytische Dehydrierung von Paraffinen oder durch Chlorierung von Paraffinen und anschließender Dehydrochlorierung der Chlorparaffine gewonnen werden (GB-PS 1 037 868).

Bei dem zuletzt genannten Verfahren werden in der Regel Paraffinschnitte, d. h. Gemische unterschiedlicher C-Zahl eingesetzt, so daß auch die erhaltenen Olefine keine einheitliche C-Zahl aufweisen.

Außerdem kommen in diesen Olefin-Gemischen natürlich alle denkbaren isomeren Formen vor.

Neben den reinen — gegebenenfalls substituierten — Olefinen können auch solche mit einem Gehalt an Paraffinen, z. B. bis zu 85 Gewichtsprozent, eingesetzt werden. Der Paraffingehalt rührt daher, daß bei der Olefinherstellung keine vollständigen Umsätze erreicht und die nicht umgesetzten Paraffine nicht oder nicht vollständig abgetrennt werden.

Nicht nur das eingesetzte Olefin, sondern auch die Art des Alkanols, das mit dem Olefin und Kohlenmonoxid umgesetzt wird, ist für das erfindungsgemäße Verfahren unkritisch. Im allgemeinen werden Alkanole mit 1 bis 10, vorzugsweise 1 bis 4 Kohlenstoffatomen, eingesetzt. Typische Vertreter aus der Gruppe der primären Aikanole sind z. B. Methanol, Ethanol, Propanol-(1) und Butanol-(1). — Die Menge des eingesetzten Alkanols beträgt im allgemeinen 1 bis 10 Mol/Mol Olefin.

Weiterhin ist es unwesentlich, welche Kobaltverbindung bei der Hydrocarboxilierung verwendet wird. Carbonyle des Kobalts, z. B. Dikobaltoctacarbonyl, sind ebenso geeignet wie carbonsaure Kobaltsalze, wie z. B. Kobaltacetat, Kobaltnaphthenat und Kobalt-2-ethyl-hexanoat, und Salze des Kobalts mit anorganischen Säuren, wie z. B. Kobaltnitrat und Kobaltsulfat. Vorzugsweise kommen solche carbonsauren Kobaltsalze zum Einsatz, deren Anionen dem Säurerest der bei der Hydrocarboxilierung gebildeten Fettsäureester entsprechen.

Die Konzentration des als Katalysator eingesetzten Kobalts liegt je nach Art des zur Umsetzung gelangenden Olefins in einem Bereich von 0,005 bis 0,2 Grammatom Kobalt pro Mol Olefin.

Schließlich sind die Reaktionsbedingungen, unter denen die Hydrocarboxilierung durchgeführt wird, für das erfindungsgemäße Verfahren nicht von Bedeutung. Im allgemeinen werden Hydrocarboxilierungsverfahren bei Temperaturen von 80 bis 300, vorzugsweise 150 bis 200 °C, und Kohlenmonoxiddrucken von 10 bis 800, vorzugsweise 100 bis 300 bar, durchgeführt.

Verfahrenskritisch für das vorliegende Verfahren ist jedoch die Verwendung eines Promotors (Cokatalysators), der als kobaltfreies Stoffgemisch bei der Aufarbeitung bestimmter Hydrocarboxilierungsgemische anfällt und der höher siedet als die in diesen Hydrocarboxilierungsgemischen enthaltenen Ester und das als Katalysatorbestandteil bei diesen Hydrocarboxilierungsreaktionen eingesetzte Pyridin und/oder nicht-ortho-substituierte Alkylpyridin. Die Hydrocarboxilierungsgemische, die das als Promotor verwendbare kobaltfreie Stoffgemisch liefern, werden durch Umsetzung von Olefinen, Kohlenmonoxid und Alkanol in Gegenwart eines Katalysators bestehend aus einer Kobaltverbindung und Pyridin und/oder nicht-ortho-substituiertem Alkyl-pyridin bei erhöhtem Druck und erhöhter Temperatur erhalten. Bezüglich der Wahl der Einsatzstoffe, Reaktandenverhältnisse und Reaktionsbedingungen unterliegt eine solche Umsetzung keinerlei besonderen Einschränkungen. Es gelten die allgemein für Verfahren dieser Art (US-PS 3 507 891 und deutsche Patentanmeldung P 29 12 489.8) einzuhaltenden Bedingungen. Sie entsprechen im wesentlichen den Bedingungen des erfindungsgemäßen Verfahrens.

Nicht kritisch für das erfindungsgemäße Verfahren ist die Art, in der die als Promotor benutzbaren Hochsieder aus dem z. B. nach einem Verfahren gemäß US-PS 3 507 891 und deutscher Patentanmeldung P 29 12 489.8 anfallenden Hydrocarboxilierungsgemisch abgetrennt werden. So können diese Hochsieder z. B. nach destillativer Entfernung unumgesetzter Einsatzstoffe sowie der als Reaktionsprodukt erhaltenen Ester aus dem dann verbleibenden kobalthaltigen Rückstand gewonnen werden. Hierfür stehen u. a. extraktive Methoden gemäß EP-OS 0 008 024 und deutscher Patentanmeldung P 29 49 939.6 und destillative Methoden, z. B. unter Benutzung eines Dünnschichtverdampfers, zur Verfügung.

Es ist jedoch auch möglich, daß man die Hochsieder beim kobalthaltigen Rückstand beläßt und den Rückstand insgesamt als Katalysatorsystem (Kobaltverbindung + Promotor) beim erfindungsgemäßen Verfahren einsetzt.

Während bei Hydrocarboxilierungsverfahren z. B. gemäß US-PS 3 507 891 und deutscher Patentanmeldung P 29 12 489.8 definierte Promotoren (Pyridin und/oder nicht-ortho-substituierte Alkylpyridine) eingesetzt werden und demzufolge auch definierte Promotor/Kobaltverhältnisse eingestellt werden können, ist dies bei Verwendung von Hochsiedern als Promotor nicht möglich, da diese aus einer Vielzahl von unbekannten Verbindungen bestehen. Bei Hydrocarboxilierungsverfahren z. B. gemäß US-PS 3 507 891 und deutscher Patentanmeldung P 29 12 489.8 wird im allgemeinen mit Promotor/Kobaltverhältnissen von 3/1 bis 25/1 (Promotor in Mol; Kobalt in Grammatom) gearbeitet; die für das erfindungsgemäße Verfahren einzuhaltenden Mengenverhältnisse können dagegen nicht von vornherein angegeben werden. Sie sind jedoch leicht durch einige orientierende Versuche zu ermitteln. Man geht dabei zweckmäßigerweise so vor, daß man Probeumsetzungen unter den Bedingungen des erfingungsgemäßen Verfahrens mit verschiedenen Mengen an Hochsiedern durchführt. Bei der Umsetzung von α-Olefinen empfiehlt es sich normalerweise mit der Hochsiedermenge zu arbeiten, für die maximaler Umsatz beobachtet wird. Das Maximum der Umsetzungsgeschwindigkeit wird bei α-Olefinen im allgemeinen von optimalen Werten für Selektivität und Linearität begleitet. Bei Verwendung von Olefinen mit innenständiger Doppelbindung liegen im allgemeinen nicht diese einfachen Zusammenhänge zwischen den angestrebten Zielgrößen vor, daher ergibt sich in diesen Fällen die Hochsiedermenge als individueller Kompromiß aus dem Verlauf von Umsetzungsgeschwindigkeit, Selektivität und Linearität als Funktion der pro Grammatom Kobalt eingesetzten Hochsiedermenge.

Im allgemeinen werden die Hochsieder in einer Menge von 2 bis 20, vorzugsweise 5 bis 15 kg, pro Grammatom Kobalt eingesetzt.

Es ist natürlich auch möglich, zusammen mit den Hochsiedern Pyridin und/oder nicht-ortho-substituierte Alkylpyridine einzusetzen, wobei 1 Mol Pyridin bzw. nicht-ortho-substituiertes Alkylpyridin

3

hinsichtlich der Promotoraktivität einer Menge von etwa 0,2 bis 1,5 kg Hochsiedern entspricht.

Mit Hilfe des erfindungsgemäßen Verfahrens, das durch die nachfolgenden Beispiele erläutert wird, gelingt es, aliphatische Carbonsäureester mit ähnlich hoher Linearität wie beim Einsatz von Pyridin und/oder nicht-orthosubstituiertem Alkylpyridin als Promotor herzustellen.

Die nach dem erfindungsgemäßen Verfahren erhaltenen Carbonsäureester sind wertvolle Zwischenprodukte für die Herstellung von z. B. Tensiden und Weichmachern.

Beispiel 1

A) Herstellung des als Promotor verwendeten Hochsieders

Ein Gemisch folgender Einsatzstoffe

1 Mol n-Dodecen (Isomerengemisch mit einem Dodecen-(1)-Anteil ≤ 1 Gewichtsprozent)
2 Mol Methanol
0,3 Mol γ-Picolin
0,03 Grammatom Kobalt (in Form eines Gemisches aus CO-Tridecanoat und Tridecansäure mit einem Kobaltgehalt von 10 %)

wird kontinuierlich in einen Rührautoklaven eingepumpt und dort unter nachfolgenden Bedingungen zur Umsetzung gebracht :

| | |
|---|---|
| Reaktionstemperature : | 185 °C |
| CO-Warmdruck : | 180 bar |
| (CO enthält 1 Vol-% $H_2$) | |
| Verweilzeit : | 1,6 h |

Der Austrag des Rührautoklaven wird kontinuierlich einem mit 8 × 8 mm Raschigringen gefüllten 1 m langen Rieselturn von oben zugeführt und mit Luft im Gegenstrom unter folgenden Bedingungen behandelt :

| | |
|---|---|
| Reaktionstemperatur : | 40 °C |
| Druck : | 1 bar |
| Flüssigkeitsdurchsatz : | 260 ml/cm² · h |
| Luft/1 1 Reaktionsaustrag : | 50 l |

Der mit Luft behandelte Reaktionsaustrag wird kontinuierlich in einer Destillationsstraße schrittweise in unumgesetztes Methanol, γ-Picolin, unumgesetztes Olefin sowie als Reaktionsprodukt gebildete Ester enthaltende Destillatfraktionen sowie einen die als Nebenprodukt anfallenden Hochsieder und das als Katalysator eingesetzte Kobalt enthaltenden Sumpf zerlegt.

Dieser Sumpf wird durch Destillation an einem Dünnschichtverdampfer

| | |
|---|---|
| Druck am Übergang : | 20 mbar |
| Heizmitteltemperatur : | 241 °C |
| Umdrehungszahl des Rotors : | 900 U/min |

soweit eingeengt, daß der hierbei anfallende Rückstand einen Kobaltgehalt von 10 % aufweist. Die Menge abdestillierten Hochsieders beträgt, bezogen auf das als Reaktionsprodukt gebildete Tridecansäuremethylestergemisch, 1,9 %.

B) Verwendung des Hochsieders als Promotor

Ein Gemisch folgender Einsatzstoffe

1 Mol n-Dodecen (Isomerengemisch mit einem Dodecen-(1)-Anteil ≤ 1 Gewichtsprozent)
2 Mol Methanol
0,03 Grammatom Kobalt (als 10 %iges CO-Tridecanoat)
420 g eines nach Beispiel 1 A hergestellten Hochsieders

wird in einem 2 1-Rührautoklaven unter folgenden Bedingungen umgesetzt :

4

| | |
|---|---|
| Reaktionstemperatur : | 185 °C |
| CO-Warmdruck : | 180 bar |
| (CO mit 1 Vol-% $H_2$) | |
| Reaktionsdauer : | 3 h |

Die gaschromatographische Analyse des Reaktionsgemisches liefert folgendes Ergebnis :

| | |
|---|---|
| Olefinumsatz : | 72 % |
| Selektivität : | 95 % |
| Anteil linearer Tridecansäuremethylester : | 74 % |

## Beispiel 2

Beispiel 1 wird mit der Ausnahme wiederholt, daß die Umsetzung in Gegenwart des Hochsieders als Promotor mit Octen-(1) bei einer Temperatur von 160 °C und einem Druck von 270 bar durchgeführt wird. Die gaschromatographische Analyse des Reaktionsgemisches liefert folgendes Ergebnis :

| | |
|---|---|
| Olefinumsatz : | 78 % |
| Selektivität : | 97 % |
| Anteil linearer Nonansäuremethylester : | 78 % |

## Beispiele 3 bis 5

Herstellung des für die Beispiele 3 bis 5 als Promotor verwendeten Hochsieders

Unter Beibehaltung der in Beispiel 1 A beschriebenen Reaktions- und Aufarbeitungsbedingungen wird unter steter Rückführung des von Hochsiedern befreiten kobalthaltigen Rückstandes als Katalysator und Wiedereinsatz der nicht umgesetzten Einsatzstoffe die in Beispiel 1 A beschriebene kontinuierliche Reaktion solange wiederholt bis der Katalysator 10 mal im Kreis geführt wurde. Die nach 10 maliger Kreisführung des Katalysators anfallenden Hochsieder (1,8 %, bezogen auf das als Reaktionsprodukt gebildete Tridecansäuremethylestergemisch) werden unter den in Tabelle 1 aufgeführten Bedingungen als Promotor eingesetzt.

## Beispiele 6 bis 8

Die Beispiele 3 bis 5 werden wiederholt mit der Ausnahme, daß in dem zur Herstellung des Hochsieders betriebenen Verfahren γ-Picolin als Promotor durch die gleiche Molmenge Pyridin ersetzt wird (siehe Tabelle 1).

(Siehe Tabelle 1 Seite 6 f.)

Tabelle 1

| Bsp. | Olefin | Alkanol | CO-Tridecanoat [10 %ig] | Promotor | Temperatur [°C] | Druck [bar] | Reaktionsdauer [h] | Umsatz [%] | Selektivität [%] | Linearität [%] |
|---|---|---|---|---|---|---|---|---|---|---|
| 3 | 1 Mol n-Dodecen[1] | 2 Mol CH₃OH | 0,03 Mol | 210 g Hochsieder | 185 | 180 | 3 | 75 | 95 | 75 |
| 4 | 1 Mol Octen-(1) | 2 Mol CH₃OH | 0,015 Mol | 210 g Hochsieder | 160 | 270 | 3 | 82 | 97 | 82 |
| 5 | 1 Mol Octen-(1) | 2 Mol CH₃OH | 0,015 Mol | 105 g Hochsieder + 14 g γ-Picolin | 160 | 270 | 3 | 80 | 96 | 83 |
| 6 | 1 Mol n-Dodecen[1] | 2 Mol CH₃OH | 0,03 Mol | 210 g Hochsieder | 185 | 180 | 3 | 73 | 94 | 74 |
| 7 | 1 Mol Octen-(1) | 2 Mol CH₃OH | 0,015 Mol | 210 g Hochsieder | 160 | 270 | 3 | 81 | 97 | 80 |
| 8 | 1 Mol Octen-(1) | 2 Mol CH₃OH | 0,015 Mol | 105 g Hochsieder + 14 g γ-Picolin | 160 | 270 | 3 | 82 | 97 | 83 |

[1] Isomerengemisch mit Dodecen-(1)-Anteil 1 Gewichtsprozent

0 047 831

**Patentansprüche**

1. Verfahren zur Herstellung von überwiegend linearen aliphatischen Carbonsäureestern durch Umsetzung von Olefinen, Kohlenmonoxid und Alkanol bei erhöhtem Druck und erhöhter Temperatur in Gegenwart eines Katalysators bestehend aus einer Kobaltverbindung und einem Promotor, dadurch gekennzeichnet, daß man als Promotor das bei der Aufarbeitung eines Reaktionsgemisches, das durch Umsetzung von Olefinen, Kohlenmonoxid und Alkanol in Gegenwart eines Katalysators, bestehend aus einer Kobaltverbindung, und Pyridin und/oder nicht-ortho-substituiertem Alkylpyridin bei erhöhtem Druck und erhöhter Temperatur erhalten wurde, anfallende kobaltfreie Stoffgemische einsetzt, das höher siedet als die als Reaktionsprodukt gebildeten Ester und das als Katalysatorbestandteil eingesetzte Pyridin und/oder nicht-ortho-substituierte Alkylpyridin.

2. Verfahren nach Anspruch 1, dadurch gekennzeichnet, daß man das kobaltfreie Stoffgemisch zusammen mit Pyridin und/oder nicht-ortho-substituiertem Alkylpyridin einsetzt.

**Claims**

1. A process for the production of a predominantly linear aliphatic carboxylic acid ester by reaction of an olefin, carbon monoxide and an alkanol at elevated pressure and elevated temperature in the presence of a catalyst comprising a cobalt compound and a promoter, characterised in that there is used as the promoter the cobalt-free mixture obtained in the working up of the reaction mixture which has been produced by the reaction of an olefin, carbon monoxide and an alkanol at elevated pressure and elevated temperature in the presence of a catalyst comprising a cobalt compound and pyridine and/or a non-ortho-substituted alkylpyridine, said cobalt-free mixture, being higher boiling than the ester formed as the product of that reaction and than the pyridine and/or non-orthosubstituted alkylpyridine used as catalyst component.

2. A process according to claim 1, characterised in that the cobalt-free mixture is used together with pyridine and/or a non-ortho-substituted alkylpyridine.

**Revendications**

1. Procédé de préparation d'esters d'acides carboxyliques aliphatiques en majeure partie linéaires, par réaction d'oléfines, de monoxyde de carbone et d'un alcanol, à pression élevée et à température élevée, en présence d'un catalyseur formé d'un composé de cobalt et d'un activeur, caractérisé par le fait que l'on utilise comme activeur le mélange de corps, exempt de cobalt, qui est obtenu lors du traitement d'un mélange réactionnel que l'on a obtenu par réaction d'oléfines, de monoxyde de carbone et d'un alcanol en présence d'un catalyseur formé d'un composé de cobalt et de pyridine et/ou d'alkyl-pyridine non substituée en ortho, à pression élevée et à température élevée, le mélange utilisé ayant un point d'ébullition supérieur à celui des esters formés comme produit de réaction et à celui de la pyridine et/ou de l'alkyl-pyridine non substituée en ortho qui est utilisée comme constituant de catalyseur.

2. Procédé selon la revendication 1, caractérisé par le fait que l'on utilise le mélange de corps exempt de cobalt en même temps que la pyridine et/ou l'alkylpyridine non substituée en ortho.